(19) **Europäisches Patentamt**

**European Patent Office**

**Office européen des brevets**

(11) Veröffentlichungsnummer: **0 257 429 B1**

(12) **EUROPÄISCHE PATENTSCHRIFT**

(45) Veröffentlichungstag der Patentschrift: **17.06.92**

(21) Anmeldenummer: **87111575.4**

(22) Anmeldetag: **10.08.87**

(51) Int. Cl.⁵: **A61B 6/04**, A61B 17/22, A61B 6/12

(54) **Lithotripsie-Arbeitsplatz.**

(30) Priorität: **22.08.86 DE 3628502**

(43) Veröffentlichungstag der Anmeldung:
**02.03.88 Patentblatt 88/09**

(45) Bekanntmachung des Hinweises auf die
Patenterteilung:
**17.06.92 Patentblatt 92/25**

(84) Benannte Vertragsstaaten:
**DE FR GB**

(56) Entgegenhaltungen:
**EP-A- 0 139 941      EP-A- 0 168 559**
**DE-A- 3 122 056      DE-A- 3 417 985**
**DE-A- 3 617 032      DE-U- 8 528 785**
**FR-A- 2 189 001**

(73) Patentinhaber: **SIEMENS AKTIENGESELL-**
**SCHAFT**
**Wittelsbacherplatz 2**
**W-8000 München 2(DE)**

(72) Erfinder: **Eichler, Jürgen, Dipl.-Ing.**
**Kilianstrasse 7**
**W-8500 Nürnberg(DE)**
Erfinder: **Oppelt, Sylvester, Dipl.-Ing. (FH)**
**Greiffenbergstrasse 51**
**W-8600 Bamberg(DE)**
Erfinder: **Grasser, Franz, Dipl.-Ing. (FH)**
**Neuwiesenstrasse 27**
**W-8551 Eggolsheim(DE)**

## Beschreibung

Die Erfindung betrifft einen Lithotripsie-Arbeitsplatz mit einem durch eine Steuervorrichtung dreidimensional verstellbaren Patientenlagerungstisch, unter dem mindestens ein Stoßwellengenerator für die Zertrümmerung von im Körper eines Patienten befindlichen und im Fokus des Stoßwellengenerators positionierten Objekten angeordnet ist, mit einer Röntgenuntersuchungseinrichtung zur Ortung dieser Objekte mit einer Röntgeneinheit, die den Patienten unter unterschiedlichen Winkeln durchstrahlen kann, und mit einer Bildverstärker-Fernsehkette für jede Strahlenrichtung.

In dem DE-U-85 28 785 ist ein derartiger Lithotripsie-Arbeitsplatz beschrieben, der zwei von einer Parkposition in eine Betriebsposition schwenkbare Stoßwellengeneratoren enthält. Zur Ortung ist der Arbeitsplatz mit einer Röntgendiagnostikeinrichtung mit zwei Röntgenstrahlenquellen zur Erzeugung zweier sich schneidender Röntgenstrahlenbündel und zwei Bildverstärker-Fernsehketten zur Darstellung der Röntgenbilder ausgerüstet. Die beiden Röntgenstrahlenbündel schneiden sich in einem Isozentrum, auf das die Stoßwellengeneratoren in Betriebsstellung fokussiert sind.

Ist das Objekt, beispielsweise ein Nieren- oder Gallenstein, durch die Röntgenuntersuchungsvorrichtung erkannt, so wird durch Verschiebung des Patientenlagerungstisches das Konkrement in das Isozentrum und damit in den Fokus des Stoßwellengenerators gebracht. Dies erfolgt im allgemeinen im Durchleuchtungsbetrieb, so daß der Patient einer unerwünscht hohen Strahlendosis ausgesetzt wird.

In der DE-A-31 22 056 ist ein Lithotripsie-Arbeitsplatz beschrieben, bei dem die Positionierung des jeweiligen Objektes im Fokus des Stoßwellengenerators mit Hilfe von in die Röntgenbilder eingeblendeten Marken erfolgt. Bei den Marken kann es sich um ortsfeste oder manuell einstellbare Marken handeln. Im ersten Fall weisen die Marken in den Röntgenbildern eine solche Position auf, daß sich ein Objekt dann im Fokus des Stoßwellengenerators befindet, wenn sein Bild in beiden Röntgenbildern mit der jeweiligen Marke zusammenfällt. Im zweiten Fall werden die Marken manuell derart eingestellt, daß sie sich in beiden Röntgenbildern mit dem Bild des Objektes decken. Anhand der Koordinaten der Marken, die abgefragt werden, wird dann der Stoßwellengenerator relativ zum Patienten derart positioniert, daß sich das Objekt im Fokus des Stoßwellengenerators befindet.

Aus der EP-A-0 139 941 ist eine Röntgendiagnostikanlage mit einem motorisch verstellbaren Patientenlagerungstisch und einer motorisch verstellbaren Primärstrahlenblende beschrieben. Dabei wird auf dem Sichtgerät einer Bildverstärker-Fernsehkette mittels eines Lichtgriffels eine diagnostisch relevante Region markiert und anhand dieser Markierung automatisch der Patientenlagerungstisch und die Primärstrahlenblende eingestellt. Dies erfolgt derart, daß sich die diagnostisch relevante Region im Zentrum des Röntgenbildes befindet und eine dem diagnostisch relevanten Bereich angepaßte Geometrie der Blendenöffnung der Primärstrahlenblende eingestellt wird.

Die Erfindung geht von der Aufgabe aus, einen Lithotripsie-Arbeitsplatz der eingangs genannten Art zu schaffen, der es ermöglicht, aufgrund von gespeicherten Röntgenaufnahmen den Patientenlagerungstisch derart zu verschieben, daß das zu behandelnde Konkrement sich im Fokus des Stoßwellengenerators befindet.

Die Aufgabe wird erfindungsgemäß dadurch gelöst, daß die Bildverstärker-Fernsehketten für zu Beginn der Behandlung erzeugte Röntgenbilder einen Speicher aufweisen, in dessen den gespeicherten Röntgenbildern entsprechende Ausgangssignale eine mittels eines den Bildverstärker-Fernsehketten zugeordneten Markengenerators erzeugbare, über deren Fernseh-Bilder manuell einstellbare, einem Raumpunkt über dem Patientenlagerungstisch entsprechende Marke eingeblendet ist, und daß der Markengenerator mit der Steuervorrichtung des Patientenlagerungstisches derart verbunden ist, daß dieser in eine Arbeitsposition steuerbar ist, in der der Raumpunkt im Fokus des Stoßwellengenerators liegt. Dadurch lassen sich zu Beginn der Behandlung zwei Röntgenbilder aus verschiedenen Ebenen speichern, in denen eine durch eine Eingabevorrichtung auf den Monitorbildern verschiebbare Marke erzeugt wird, die auf die georteten Objekte eingestellt wird. Durch die entsprechende Nachführung des Patientenlagerungstisches wird erreicht, daß dieser derart in eine Arbeitsposition verschoben wird, daß sich das geortete und derart markierte Objekt im Fokus des Stoßwellengenerators befindet.

Die derart gefundene Arbeitsposition läßt sich jederzeit erneut ohne weitere Durchleuchtung auffinden, wenn der Markengenerator einen Speicher für die Koordinaten der Arbeitsposition aufweist. Eine hochauflösende Röntgenaufnahme zur Zwischenbefundung kann erstellt werden, wenn die Steuervorrichtung derart ausgebildet ist, daß der Patientenlagerungstisch aus der eingegebenen, gespeicherten Arbeitsposition derart verfahrbar ist, daß eine Aufnahmevorrichtung aus einer Parkstellung in ihre Aufnahmestellung unterhalb des Patienten verfahrbar ist, und daß nach erfolgter Aufnahme die Aufnahmevorrichtung in ihre Parkstellung und der Patientenlagerungstisch wieder in die gespeicherte Arbeitsposition verfahrbar sind. Dadurch wird erreicht, daß die Röntgenaufnahmevorrichtung ohne Störung durch den Stoßwellengenerator oder die Bildverstärker in ihre optimale Aufnahmestellung gefahren werden kann und nach erfolgter Aufnahme der Patientenlagerungstisch zur weiteren Behandlung in seine Arbeitsposition verfahrbar ist.

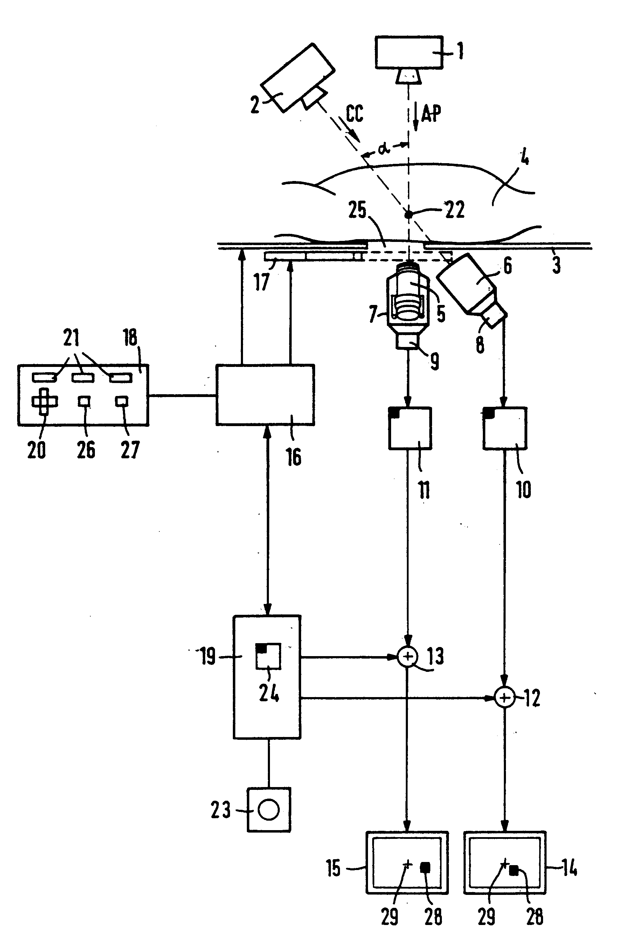

Die Erfindung ist nachfolgend anhand eines in der Zeichnung dargestellten Ausführungsbeispieles näher erläutert.

In der Figur ist ein Lithotripsie-Arbeitsplatz mit einer Röntgendiagnostikeinrichtung dargestellt, die zwei Röntgenröhren 1 und 2 aufweist, die Röntgenstrahlenbündel erzeugen, die einen auf einem Patientenlagerungstisch 3 befindlichen Patienten 4 durchdringen. Unterhalb des Patientenlagerungstisches 3 sind wenigstens ein Stoßwellengenerator 5 und zwei Röntgenbildverstärker 6 und 7 angeordnet. Die Röntgenröhre 1 und der Röntgenbildverstärker 7 können dabei derart angeordnet sein, daß der Zentralstrahl des Röntgenstrahlenbündels der Röntgenröhre 1 senkrecht auf den Patienten 4 fällt (a.p.-Projektion). Die Röntgenröhre 2 und der Röntgenbildverstärker 6 sind beispielsweise derart schräg angeordnet, daß der Zentralstrahl der Röntgenröhre 2 den Zentralstrahl der Röntgenröhre 1 im Isozentrum innerhalb des Patienten 4 unter einem Winkel alpha von beispielsweise 45° schneidet (c.c.-Projektion). Dadurch erhält man Durchleuchtungsbilder aus zwei verschiedenen Projektionsrichtungen, so daß man den Patienten 4 durch den Patientenlagerungstisch 3 derart dreidimensional verschieben kann, daß sich ein Objekt 22, beispielsweise ein Nieren- oder Gallensein, in dem Isozentrum und damit im Fokus des Stoßwellengenerators 5 befindet.

In dem Patientenlagerungstisch 3 ist eine Öffnung 25 angebracht, durch die der Stoßwellengenerator 5 aus seiner Parkposition in die Betriebsstellung geschwenkt werden kann. Mit der Schwenkung wird gleichzeitig der Fokus des Stoßwellengenerators 5 auf das Isozentrum ausgerichtet, so daß in der Betriebsstellung die Stoßwellenbehandlung erfolgen und das Objekt 22, beispielsweise ein Nieren-oder Gallenstein, zertrümmert werden kann.

Die Ausgangssignale der an den Röntgenbildverstärkern 6 und 7 angekoppelten Fernsehkameras 8 und 9 können in zwei Bildspeichern 10 und 11 eingelesen werden. Die Ausgangssignale der Bildspeicher 10 und 11 sind mit den Eingängen von Additionsstufen 12 und 13 verbunden, an denen je ein Monitor 14 und 15 zur Wiedergabe der Röntgenbilder angeschlossen ist.

Nach erfolgter Durchleuchtung durch die Röntgenröhren 1 und 2 werden die Röntgenstrahlenbilder von den Bildwandlern, den Bildverstärkern 6 und 7 sowie den angekoppelten Fernsehkameras 8 und 9, in Bildsignale umgewandelt, die in den Bildspeichern 10 und 11 eingespeichert werden. Den Röntgenstrahlenbildern wird, wie noch erläutert, in den Additionsstufen 12 und 13 ein Signal zugemischt, so daß auf den Monitoren 14 und 15 die Röntgenstrahlenbilder mit einer Einblendung dargestellt werden.

Zur dreidimensionalen Verschiebung des Patientenlagerungstisches 3 ist dieser mit einer Steuervorrichtung 16 für dessen motorische Verstellung verbunden. Eine unter dem Patientenlagerungstisch 3 angeordnete Röntgenaufnahmevorrichtung, beispielsweise eine Kassettenlade 17, ist ebenfalls mit der Steuervorrichtung 16 verbunden und läßt sich bei Bedarf von ihrer dargestellten Parkstellung in die gestrichelt dargestellte Aufnahmestellung verfahren. Die Steuervorrichtung 16 ist weiterhin mit einem Bedienpult 18 zur Eingabe der erforderlichen Funktionen verbunden. So läßt sich beispielsweise der Patientenlagerungstisch 3 durch die Betätigung von Einstellmitteln 20 dreidimensional verschieben, wobei die aktuelle Position des Patientenlagerungstisches 3 durch Anzeigemittel 21 für die drei Raumkoordinaten x, y und z auf dem Bedienpult 18 erkennbar ist.

An der Steuervorrichtung 16 ist weiterhin ein Markengenerator 19 angeschlossen, der mit den Additionsstufen 12 und 13 zur Einblendung einer Marke 28 in die Bilder der Monitore 14 und 15 verbunden ist. Über eine Eingabevorrichtung 23 läßt sich die in den Monitorbildern eingeblendete Marke 28 verschieben. Dies kann beispielsweise dadurch erfolgen, daß zuerst die Marke 28 durch die Eingabevorrichtung 23 auf dem Bild des Monitors 15 verschoben wird, so daß die Kennzeichnung des Objektes 22 in x-und y-Richtung (horizontale Richtungen) festliegt. Anschließend wird die Eingabevorrichtung umgeschaltet, so daß nunmehr die Marke 28 auf dem Bild des Monitors 14 derart verschoben wird, daß sie sich mit der Abbildung des Objektes 22 deckt, so daß hieraus die Lage des Objektes in z-Richtung (vertikale Richtung) bestimmbar ist. Sind nun die Marken 28 mit der Abbildung des Objektes 22 in Deckung gebracht worden, so werden die aktuellen Koordinaten der Marken 28 in einem Speicher 24 abgelegt, der beispielsweise im Markengenerator 19 angeordnet sein kann. Aus den Differenzen dieser gespeicherten Daten und den Koordinaten des Isozentrums, das sich, wie durch ein Fadenkreuz 29 dargestellt, auf den Monitorbildern in der Mitte befindet, wird ein Signal berechnet und der Steuervorrichtung 16 zugeführt. Nun wird der Patientenlagerungstisch 3 beispielsweise durch Betätigung des Auslöseknopfes 26 am Bedienpult 18 durch die Steuervorrichtung 16 derart verschoben, daß sich das Objekt 22 im Isozentrum und somit im Fokus des Stoßwellengenerators 5 befindet. Gleichzeitig werden die Koordinaten dieser Position des Patientenlagerungstisches 3 als Arbeitsposition in den Speicher 24 eingelesen.

Die Eingabevorrichtung 23 kann, wie dargestellt, aus einem sogenannten "Trackball", einer Rollkugel, gebildet sein. Sie kann aber auch aus einem Lichtgriffel, aus den drei Raumkoordinaten zugeordneten Potentiometern oder einer Widerstandsfolie bestehen.

Soll nun nach erfolgter Behandlung zur Zwischenkontrolle oder zur Endbefundung eine Röntgenfilmaufnahme erstellt werden, so wird durch Betätigung beispielsweise des Auslöseknopfes 27 der Patientenlagerungstisch 3 aus der Arbeitsposition verfahren, so daß die Kassettenlade 17 von ihrer dargestellten Parkstellung in ihre gestrichelt dargestellte Aufnahmestellung unterhalb des Patienten 4 verschoben werden kann. Anschließend wird dann die Röntgenröhre 1 ausgelöst. Ist die Aufnahme beendet, wird die Kassettenlade 17 wieder in ihre Parkstellung und der Patientenlagerungstisch aufgrund der in dem Speicher 24 enthaltenen Koordinaten in seine Arbeitsposition zurückgefahren, so daß je nach Bedarf die Behandlung fortgesetzt werden kann.

Durch diese erfindungsgemäße Ausführung des Lithotripsie-Arbeitsplatzes läßt sich die Strahlenbelastung des Patienten gering halten, da die Einstellung des Patientenlagerungstisches nicht unter kontinuierlicher Durchleuchtung erfolgen muß.

**Patentansprüche**

1. Lithotripsie-Arbeitsplatz mit einem durch eine Steuervorrichtung (16) dreidimensional verstellbaren Patientenlagerungstisch (3), unter dem mindestens ein Stoßwellengenerator (5) für die Zertrümmerung von im Körper eines Patienten (4) befindlichen und im Fokus des Stoßwellengenerators (5) positionierten Objekten (22) angeordnet ist, mit einer Röntgenuntersuchungseinrichtung (1, 2, 6 bis 15) zur Ortung dieser Objekte (22) mit einer Röntgeneinheit (1, 2), die den Patienten (4) unter unterschiedlichen Winkeln durchstrahlen kann, und mit einer Bildverstärker-Fernsehkette (6 bis 15) für jede Strahlenrichtung, **dadurch gekennzeichnet,** daß die Bildverstärker-Fernsehketten (6 bis 15) für zu Beginn der Behandlung erzeugte Röntgenbilder einen Speicher (10, 11) aufweisen, in dessen den gespeicherten Röntgenbildern entsprechende Ausgangssignale eine mittels eines den Bildverstärker-Fernsehketten (6 bis 15) zugeordneten Markengenerator (19) erzeugbare, über deren Fernseh-Bilder manuell einstellbare, einem Raumpunkt über dem Patientenlagerungstisch (3) entsprechende Marke (28) einblendbar ist, und daß der Markengenerator (19) mit der Steuervorrichtung (16) des Patientenlagerungstisches (3) derart verbunden ist, daß dieser in eine Arbeitsposition steuerbar ist, in der der Raumpunkt im Fokus des Stoßwellengenerators (5) liegt.

2. Lithotripsie-Arbeitsplatz nach Anspruch 1, **dadurch gekennzeichnet**, daß der Markengenerator (19) einen Speicher (24) für die Koordinaten der Arbeitsposition aufweist.

3. Lithotripsie-Arbeitsplatz nach Anspruch 2, **dadurch gekennzeichnet**, daß die Steuervorrichtung (16) derart ausgebildet ist, daß der Patientenlagerungstisch (3) aus der eingegebenen, gespeicherten Arbeitsposition derart verfahrbar ist, daß eine Aufnahmevorrichtung (17) aus einer Parkstellung in ihre Aufnahmestellung unterhalb des Patienten (4) verfahrbar ist, und daß nach erfolgter Aufnahme die Aufnahmevorrichtung (17) in ihre Parkstellung und der Patientenlagerungstisch (3) wieder in die gespeicherte Arbeitsposition verfahrbar sind.

**Claims**

1. A lithotripsy work station having a patient support table (3) which can be adjusted in three dimensions by means of a control device (16), under which patient support table there is arranged at least one shock wave generator (5) for disintegrating objects (22) located in the body of a patient (4) and positioned at the focus of the shock wave generator (5), having an X-ray examination apparatus (1, 2, 6 to 15) for locating these objects (22) with an X-ray unit (1, 2) which can radiograph the patient (4) at various angles, and having an image intensifier-video chain (6 to 15) for each direction of radiation,
**characterised in that** the image intensifier-video chains (6 to 15) have a memory (10, 11) for X-ray images generated at the start of treatment, with a mark (28) being capable of insertion into the output signals of this memory which correspond to the stored X-ray images, which mark can be generated by means of a mark generator (19) assigned to the image intensifier-video chains (6 to 15) and can be adjusted manually on the video images therefrom and corresponds to a point in space above the patient support table (3), and in that the mark generator (19) is connected to the control device (16) of the patient support table (3) in such a way that the said table can be directed into a working position in which the point in space is located at the focus of the shock wave generator (5).

2. A lithotripsy work station according to claim 1, characterised in that the mark generator (19) has a memory (24) for the coordinates of the working position.

3. A lithotripsy work station according to claim 2, characterised in that the control device (16) is constructed so that the patient support table (3) can be moved from the entered and stored working position in such a way that an exposure device (17) can be moved from a standby position into its exposure position under the patient (4), and in that, after exposure has taken place, the exposure device (17) can be moved back into its standby position and the patient support table (3) can be moved back into the stored working position.

**Revendications**

1. Poste de traitement de lithotritie comportant un plateau formant couchette pour patient (3), qui est réglable dans trois dimensions par l'intermédiaire d'un dispositif de commande (16) et au-dessous duquel est disposé au moins un générateur d'ondes de choc (5) servant à désintégrer des objets (2) situés dans le corps d'un patient (4) et positionnés au foyer du générateur d'ondes de choc (5), et un dispositif de radiodiagnostic (1,2,6 à 15) servant à localiser ces objets (22) et comportant une unité radiologique (1,2), qui peut irradier le patient (4) sous des angles différents, et une chaîne de télévision à amplificateur de brillance (10 à 15) pour chaque direction de rayonnement, caractérisé par le fait les chaînes de télévision à amplificateur de brillance (6 à 15) comportent, pour des radiographies produites au début du traitement, une mémoire (10,11), qu'une marque (28), qui peut être produite au moyen d'un générateur de marques (19) associé aux chaînes de télévision à amplificateur de brillance (6 à 15), réglable manuellement au moyen de leurs images de télévision et correspondant à un point de l'espace au-dessus du plateau formant couchette pour patient (3), peut être insérée dans les signaux de sortie de la mémoire qui correspondent aux radiographies mémorisées, et que le générateur de marques (19) est raccordé au dispositif de commande (16) du plateau formant couchette pour patient (3) de telle sorte que ce plateau peut être commandé dans une position de travail, dans laquelle le point dans l'espace est situé au foyer du générateur d'ondes de choc.

2. Poste de traitement de lithotritie suivant la revendication 1, caractérisé par le fait que le générateur de marques (19) comporte une mémoire (24) pour les coordonnées de la position de travail.

3. Poste de traitement de lithotritie suivant la revendication 2, caractérisé par le fait que le dispositif de commande (16) est agencé de telle sorte que le plateau formant couchette pour patient (3) peut être déplacé à partir d'une position de travail introduite et mémorisée, de sorte qu'un dispositif d'enregistrement (17) peut être déplacé depuis une position de rangement, dans sa position d'enregistrement au-dessous du patient (4), et qu'après l'obtention d'une prise de vue, le dispositif d'enregistrement (17) peut être ramené dans sa position de repos et que le plateau formant couchette pour patient (3) peut être ramené dans la position de travail mémorisée.